# EUROPEAN PATENT APPLICATION

(11) **EP 1 935 381 A2**
(43) Date of publication of application: **25.06.2008**
(21) Application number: 07123310.0
(22) Date of filing: 17.12.2007
(51) Int. Cl.: A61F 5/37

(54) **Immobilization support device**

(30) Priority: 22.12.2006 US 615375
(71) Applicant: Kendrick EMS, Inc., Mooresville, North Carolina 28117 (US)
(72) Inventor: Kendrick, Richard L., Mooresville, NC 28117 (US)
(74) Representative: Kayser, Martin

(57) **Abstract**

The invention is a support device (20) that cradles and supports the head of an individual while distributing pressure acting on the head. The invention may be used in conjunction with a spine board (50) or other cervical immobilization device to maintain the cervical spine in neutral alignment and assist in immobilization of the individual.

## Description

### FIELD OF THE INVENTION

The present invention relates generally to an immobilization support and alignment device for use in the transportation of injured persons.

### BACKGROUND

An important aspect of the job of emergency medical personnel is to safely transport injured persons from the scene of the injury to a hospital. To accomplish this task, a wide variety of equipment may be used to ensure that injuries sustained to the patient's head or neck are not aggravated during travel to the hospital. The kinds of equipment employed include stretchers, spine boards, splints, extrication collars and jackets, and the like.

The equipment for transporting patients must be rigid to provide the necessary support, and the patient must be tightly secured to the equipment to restrict any unnecessary movement. The constraint of a patient against rigid equipment, however, often results in undue pressure being placed on the tissue of the transported persons. Specifically, immobilization of a patient's head can place a great deal of pressure on the occiput, the bone forming the back of the skull. As a result, immobilization on rigid spine boards can be painful and may produce tissue-interface pressure high enough to result in the development of pressure ulcers (*i.e.*, "bedsores"), ischemia (*i.e*., restriction of blood supply), or even necrosis of the tissue (*i.e.*, death of living tissue).

To combat this problem of pressure-caused tissue damage, a variety of padding and cushioning is used to distribute the pressure caused by the body's contact with the hard equipment during immobilization. Each solution currently in use has its drawbacks, though. Large cushions such as air mattresses cradle the patient but are bulky and take up precious space in emergency vehicles. Towels or blankets may provide some cushioning, but these items must be rolled or folded to address each individual situation. Small, flat pads provide some cushioning to relieve the pressure, but they do not help to secure the patient. Devices specially designed to support and immobilize a patient's head are useful, but serve only that purpose. Unfortunately, such devices, even in their intended use, fail to provide means for adjusting the thickness of the padding to account for different body shapes.

As a result of the deficiencies in each of the devices currently in use, there exists a need for a device that readily provides relief from the pressure caused by immobilization while occupying a minimum of storage space, and that also assists in securing the patient during transport.

### SUMMARY OF THE INVENTION

In one aspect, the invention is a support device for distributing the pressure applied by a surface against a person's head. This device may be used in conjunction with a spine board or other cervical immobilization device to maintain the cervical spine in neutral alignment while assisting in immobilization.

In another aspect, the invention is a device for immobilizing the head of an individual while distributing the force applied by the individual's head against a surface. The device of this embodiment includes a compressible support having an indentation for supporting or cradling the head of the individual.

In yet another aspect, the invention is a device for immobilizing the head of an individual that can be stored in a compressed shape until used. The device of this embodiment includes a non-stick, compressible support having an indentation for supporting the head of the individual and an inflation seal that prevents the introduction or removal of air from the interior of the support.

The foregoing and other objects and advantages of the invention and the manner in which the same are accomplished will become clearer based on the following detailed description taken in conjunction with the accompanying drawings.

### BRIEF DESCRIPTION OF THE DRAWINGS

Figure 1 is a perspective view of one embodiment of the invention depicting a first support, a fastening strap, and a head strap sealed within an enclosure.

Figure 2 is a perspective view of the bottom of one embodiment of the invention showing the first support, the fastening strap, a fastening device, and the head strap.

Figure 3 is a perspective view of the top of one embodiment of the invention showing the first support, the fastening strap, and an inflation seal.

Figure 4 is a partial cross-sectional perspective view of one embodiment of the invention depicting a section of the first support containing the inflation seal.

Figure 5 is a cross-sectional view of one embodiment of the invention depicting the positioning of a patient's head within an indentation of the first support.

Figure 6 is a perspective view of one embodiment of the head strap that may be used in conjunction with the first support to secure a patient's head.

Figure 7 is a partial perspective view of the invention in one embodiment depicting the positioning of the first support, the fastening strap, and the fastening device with respect to a spine board and patient.

Figure 8 is a partial cross-sectional view of one embodiment of the invention illustrating the positioning of the first support and fastening strap with respect to a spine board and patient.

Figure 9 is a perspective view of the invention in one embodiment depicting the positioning of the first support, fastening strap, fastening device, and a head strap with respect to a spine board and the patient.

Figure 10A is a side elevation view of one embodiment of the invention depicting two stacked supports serving to elevate the head of an adult patient from a spine board.

Figure 10B is a side elevation view of one embodiment of the invention depicting multiple stacked supports serving to elevate the torso of a child patient from a spine board.

### DETAILED DESCRIPTION

One purpose of the present invention is to avoid the infliction of tissue damage to injured persons immobilized for transport to a medical care facility. The force required to effectively restrict the patient's movement causes pressure to the tissue at the point where the patient's body contacts the immobilization equipment. The present invention addresses this issue by supporting the patient's body at more than a single contact point. This design provides better patient comfort as well as adding stability. A secondary but likewise important problem addressed by this invention is the ability to maintain the immobilized patient's spine in proper neutral alignment.

The terminology used herein is for the purpose of describing particular embodiments only and is not intended to be limiting of the invention. As used herein, the term "and/or" includes any and all combinations of one or more of the associated listed items. As used herein, the singular forms "a," "an," and "the" are intended to include the plural forms as well as the singular forms, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one having ordinary skill in the art to which this invention belongs. It will be further understood that terms, such as those defined in commonly used dictionaries, should be interpreted as having a meaning that is consistent with their meaning in the context of the relevant art and the present disclosure and will not be interpreted in an idealized or overly formal sense unless expressly so defined herein.

For ease of discussion, the apparatus will be described with reference to emergency medical transportation of injured persons. Those having ordinary skill in the art will recognize that the invention is applicable to a variety of medical applications other than emergency immobilization and transportation. Those having ordinary skill in the art will recognize that the invention is applicable to other medical uses, and shall not be so limited.

In one embodiment, the invention is a support device 20 that may be used in conjunction with a spine board 50 or other cervical immobilization device to relieve the pressure caused by physical restraints while assisting in immobilization of the upper torso and maintaining the cervical spine in neutral alignment. The invention provides a first support 21 having a first external surface 22, a second external surface 23, an opening 24 defined by the first support 21, and a sheet member 27. The first support 21 defines an interior and has a thickness such that pressure applied by the patient's head is distributed from the point of contact, reducing the concentration of pressure. The sheet member 27 traverses the opening 24 in the first support 21, providing a surface within the opening 24.

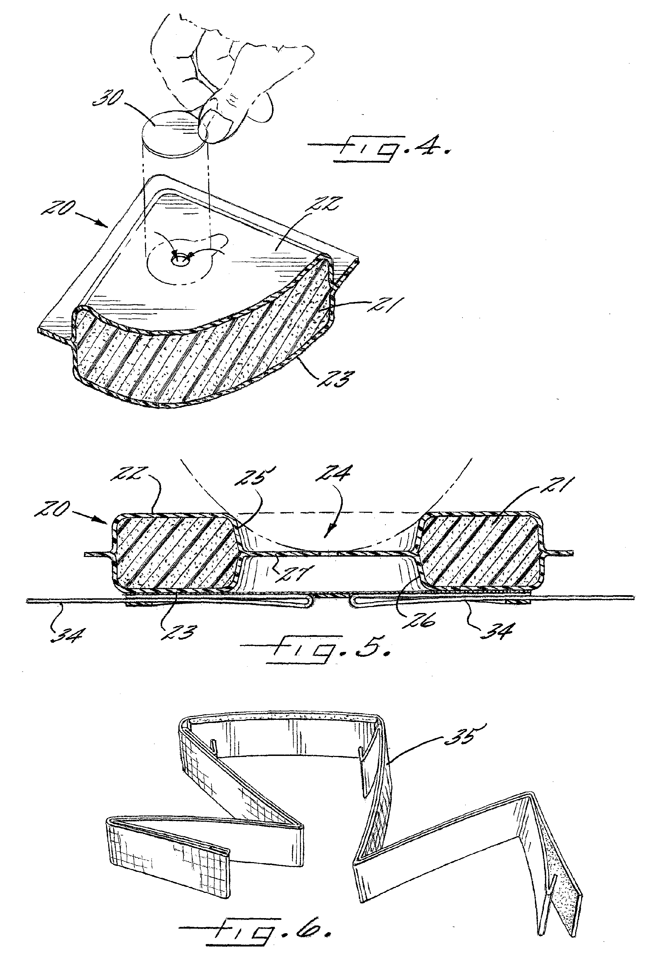

The sheet member 27 may be secured within the opening at any level throughout the thickness of the first support 21. For example, the sheet member 27 may be flush with the first external surface 22. Alternatively, the sheet member 27 may be positioned such that it is recessed with respect to the first external surface 22, thereby defining a first indentation 25. Similarly, if the sheet member 27 is recessed with respect to the second external surface 23, a second indentation 26 may be defined. In the embodiment illustrated in Figure 5, the sheet member 27 traverses the opening 24 approximately midway through the thickness of the first support 21. In this way, the sheet member 27 and the edges of the first support 21 surrounding the opening 24 cradle the patient's head. The cradling design of the first support 21 also serves to aid the immobilization of the patient as the patient's head rests within the indentation 25, restricting lateral movement of the patient's head.

Figure 5 shows a cross-section of one embodiment of the first support 21, wherein a ring of cushioning material defines the opening 24, and the sheet member 27 secured to the first support 21 in the middle of the opening 24 (i.e., midway through the thickness of the first support) defines the first indentation 25. The sheet member 27 provides support for the patient's head in addition to support provided by the edges of the ring. The first and second external surfaces 22, 23 comprise a cover layer, which may be made of a non-stick material such as surgical rubber.

The first support 21 is designed such that the use of this embodiment of the invention maintains the prone position of the patient's head relative to the rest of the body. As previously stated with respect to Figure 5, the patient's head rests within the opening 24 of the first support 21, supported by the sheet member 27. In this configuration, the patient's head is not elevated by the full thickness of the first support 21 as it rests on the sheet member 27 within the opening 24. Advantageously, the compression of the first support 21 under pressure from the head further diminishes the elevation of the patient's head relative to the spine board 50 and the rest of the body. If it is desired to elevate the patient's head, additional supports 41 may be stacked together with the first support 21 to reach the desired support height.

In another embodiment, a first support 21 having a first external surface 22, a second external surface 23, and a sheet member 27 is provided. In this embodiment, the first support 21 defines a first indentation 25 in the first external surface 22. The first support 21 may also define a second indentation 26 in the second external surface 23 of the first support 21. The second indentation 26 is correspondingly aligned with the first indentation 25 and is separated from the first indentation 25 by the sheet member 27. This embodiment may likewise serve to cradle and support the patient's head as it rests within the first indentation 25. Further, as the first and second indentations 25, 26 need not extend throughout the entire thickness of the first support 21, this embodiment may provide additional cushioning to aid in the support of the patient's head.

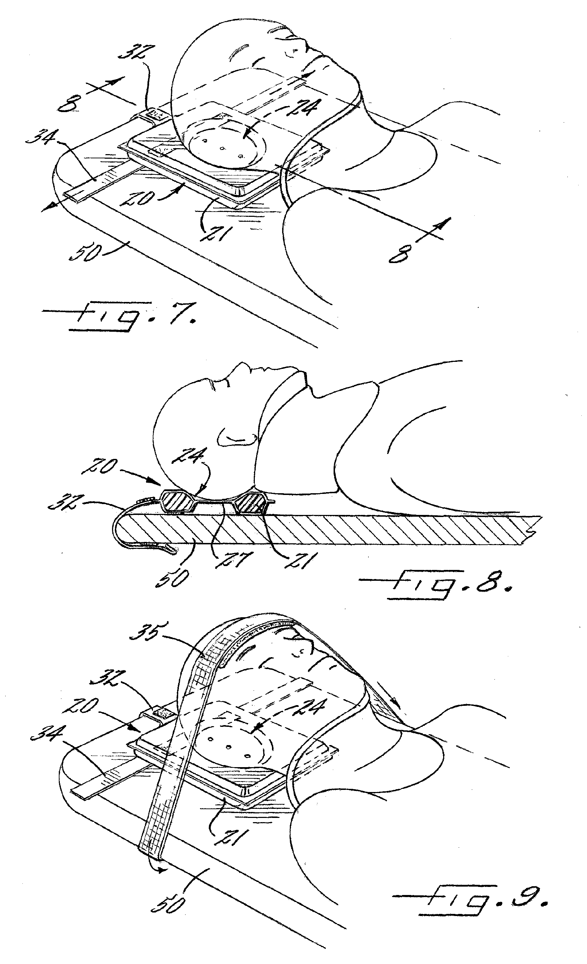

As illustrated in Figures 7 through 9, a fastening strap 32 attached to the first support 21 may be used to hold the support in place while the patient is secured to the spine board 50. The fastening strap 32 may be held by the emergency medical personnel to position the first support 21 for proper alignment. Alternatively, the fastening strap 32 may include a fastener 33 to secure the first support 21 to the spine board 50. The fastener 33 may be a snap, button, adhesive, hook-and-loop fastener, or any other kind of fastener known in the art.

To further assist in aligning the first support 21 and securing it to the spine board 50, a fastening device 34 may also be provided. In the embodiment illustrated in Figure 7, a segment of double-sided adhesive tape may be used as the fastening device 34. One surface of the tape is secured to the first support 21. The other surface may be covered by a set of plastic tabs until the device is used. When the first support 21 is placed in the desired position on the spine board 50, the tabs are pulled to expose the adhesive surface of the tape, securing the first support 21 to the spine board 50 surface. Although double-stick tape serves as the fastening device 34 in the illustrated embodiment, any suitable fastening means may be used.

Figure 7 depicts one embodiment of the invention in use. The first support 21 is positioned beneath the patient's head so that the occiput rests within the first indentation 25. The fastening strap 32 secures the first support 21 against the spine board 50, and the first support 21 is further secured to the spine board 50 by pulling the tabs on the double-sided tape fastening device 34 to expose the adhesive. As shown in cross-sectional view of the invention in Figure 8, the sheet member 27 secured within the first indentation 25 cradles the patient's head, distributing the applied pressure so that no single point on the patient's skull is subjected to a significant amount of force.

To aid in immobilizing an injured person on a spine board 50, a head strap 35 may be used in conjunction with the support device 20. As shown in Figure 6, the head strap 35 may include a length of adhesive tape having a piece of cushioning attached to the center of the length at a point of contact between the patient's head and the head strap 35. When ready for use, the adhesive of the head strap 35 is exposed so that it may be secured to the spine board 50. Figure 9 depicts the application of a head strap 35 in combination with the immobilization support device 20. The head strap 35 is secured tightly to the spine board 50 so that the patient's head is pinned between the head strap 35 and the first support 21.

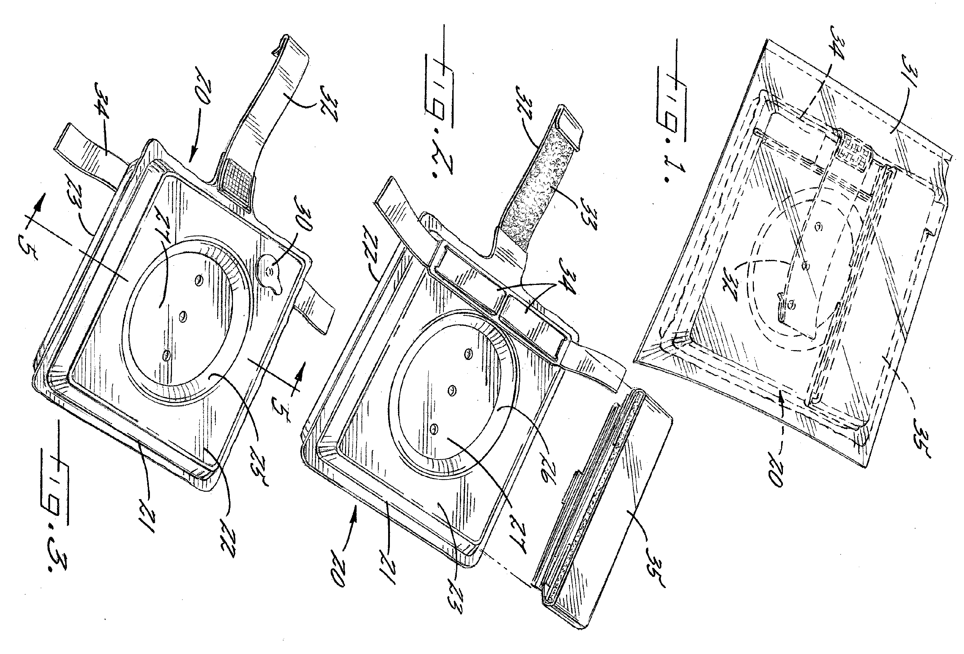

To address concerns over storage space for emergency medical equipment, the first support 21 may be constructed from a compressible material, such as foam rubber. The first support 21 may then be vacuum sealed to remove excess air from the foam. The first support 21 may also be stored within a sealed container, such as a polymeric bag, to maintain the first support 21 in a sterile condition until it is used. Figure 1 shows an embodiment of the invention sealed within such a polymeric bag 31 for storage. An inflation seal 30 on the first support 21 prevents the introduction of air into the interior of the first support 21. The inflation seal 30 also inhibits the removal of air from the interior of the first support 21 when the first support 21 is in an expanded shape upon inflation. The inflation seal 30 thus maintains the first support 21 in this compressed shape, preserving the integrity of the device and reducing its size for storage. As shown in Figure 4, upon operational use, the inflation seal 30 is disengaged to allow air to re-enter the interior, thereby expanding the first support 21 to a maximum thickness.

Figure 2 depicts one view of one embodiment of the invention upon removal from its packaging. As illustrated, the second external surface 23 of the first support 21 carries the fastening strap 32 and the fastening device 34. A head strap 35 may also be included in the package. Figure 3 shows the first external surface 22 of the first support 21 in this embodiment.

The invention also serves to maintain the patient's cervical spine in proper neutral alignment. Although the use of a single first support 21 maintains the prone position of the patient's head, additional supports 41 may be stacked on top of the first support 21 to achieve the desired thickness of support material. The additional supports 41 likewise have a first and second external surface, at least one indentation, and a sheet member secured to the additional support 41 and positioned within the first indentation. The indentations of the first and additional supports 21, 41 may be aligned to cradle the patient's head.

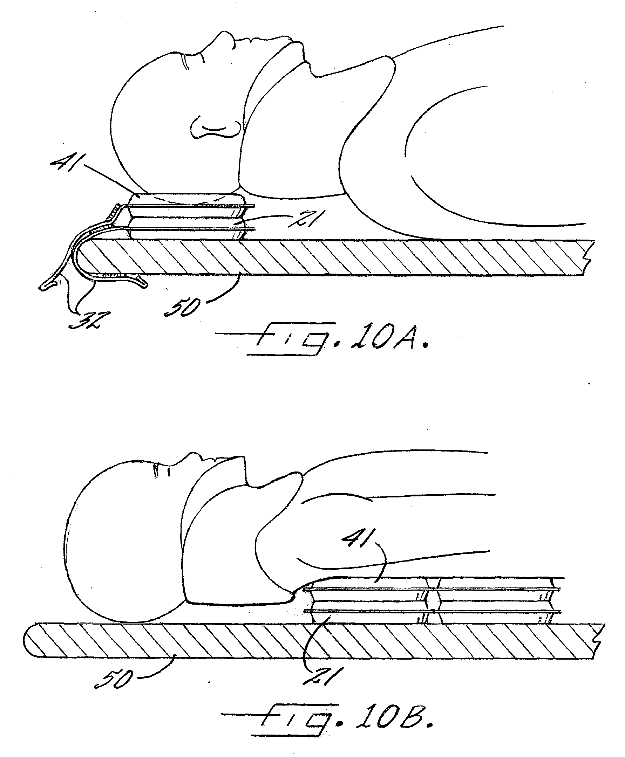

Additional thickness may be required where the patient's head and torso are not in linear alignment, and thus positioning the patient on a flat surface results in either compression or extension of the spine. As shown in Figure 10A, a typical adult may require elevation of the head relative to the torso to achieve neutral alignment.
If the emergency medical personnel hold the patient's head at the proper height to achieve this alignment, a gap between the patient's head and the spine board 50 is thus created. One or more additional supports 41 are necessary to fill this gap and maintain correct spine alignment. The stacked first and additional supports 21, 41 may be secured to each other and to the adjacent surface using the fastening strap 32 of each device.

Alternatively, as shown in Figure 10B, many young children exhibit the opposite relationship between their head and torso in neutral alignment. Because of the large size of a child's head relative to his body, placement on a flat spine board 50 may cause flexion on the spine. One or more additional supports 41 may thus be placed beneath the patient's back to elevate his torso relative to his head. The additional supports 41 should be placed on the spine board 50 prior to positioning the patient on the spine board 50. If the stacking of multiple supports overcompensates for the natural tendency for such a patient to exhibit spinal compression while on a flat surface, resulting instead in extension of the spine, additional supports may be used as described above to elevate the patient's head to achieve neutral alignment.

Finally, although the invention is disclosed as a device for use in immobilizing a patient's head, the invention is capable of serving a variety of additional purposes. As described above, the invention may be used to provide support or cushioning under a patient's back. Likewise, the invention may be used to support a patient's heels or to provide a cushion between a patient's knees.
The uses described in this application are in no way a complete disclosure of every circumstance in which this device may be useful.

## Claims

1. A support device for the head comprising:
a first support having a first external surface and a second external surface, said first support defining an interior and having a thickness sufficient to support a force applied to said first support;
an opening defined by said first support and extending through said first support; and
a sheet member secured to said first support and positioned to traverse said opening;
wherein said first support and said sheet member distribute pressure applied by the force about said first support.

2. A support device according to Claim 1
wherein said first and second external surfaces are constructed from a non-stick material.

3. A support device according to Claim 1
wherein said first support is formed from a compressible material.

4. A support device according to Claim 1
wherein said sheet member traverses said opening at a position recessed with respect to said first external surface.

5. A support device according to Claim 1 further comprising an inflation seal positioned on said first support, said inflation seal preventing the introduction or removal of air from the interior of said first support.

6. A support device according to Claim 1 further comprising a sealed polymeric bag, wherein said support is substantially contained within an interior space defined by said sealed polymeric bag.

7. A support device according to Claim 1 further comprising a fastening strap attached to said first support, said fastening strap having a fastener for securing said first support to a surface.

8. A support device according to Claim 1 further comprising a fastening device attached to said first support, said fastening device for securing said first support to a surface.

9. A support device according to Claim 1 further comprising:
at least one additional support having a first external surface and a second external surface;
wherein said at least one additional support is stacked on top of said first support.

10. A support device according to Claim 9
wherein said first support and said at least one additional support are secured to one another and an adjacent surface.

11. A device for immobilizing the head of an individual comprising:
a first support formed from a compressible material and having a first external surface and a second external surface, said first support having a thickness sufficient to support the head of an individual;
a first indentation defined by said first external surface, said first indentation for receiving the head of an individual; and
a second indentation defined by said second external surface, said second indentation correspondingly aligned with said first indentation;
wherein said first support distributes a force applied by the head to an adjacent surface supporting said first support and alleviates pressure acting against the head.

12. An immobilizing device according to Claim 11 further comprising a sheet member secured to said first support and separating said first and second indentations, said sheet member supporting at least a portion of the head.

13. An immobilizing device according to Claim 11 further comprising an inflation seal positioned on said first support, said inflation seal preventing the introduction or removal of air from said compressible material of said first support.

14. An immobilizing device according to Claim 13 wherein said compressible material expands to a maximum thickness when said inflation seal is disengaged.

15. An immobilizing device according to Claim 11 further comprising a fastening strap attached to an edge of said first support, said fastening strap having a fastener for securing said first support to the adjacent surface.

16. An immobilizing device according to Claim 11 further comprising a fastening device attached to one of said first or second external surfaces of said first support, said fastening device for securing said first support to the adjacent surface.

17. An immobilizing device according to Claim 11 further comprising at least one additional support defining a first indentation for receiving portions of the head, said at least one additional support secured to said first support and the adjacent surface such that said first and second indentations of said first support and said first indentation of said at least one additional support are aligned.

18. A device for immobilizing the head of an individual comprising:
a first support formed from a compressible material and having a first external surface and a second external surface, said first and second external surfaces surrounding said compressible material, said first support having a thickness sufficient to support the head of an individual;
a first indentation defined by said first external surface, said first indentation for receiving the head of an individual;
a second indentation defined by said second external surface, said second indentation correspondingly aligned with said first indentation;
a sheet member secured to said first support and separating said first and second indentations, said sheet member supporting at least a portion of the head; and
an inflation seal positioned on said first support, said inflation seal preventing the introduction or removal of air from said compressible material of said first support.

19. An immobilizing device according to Claim 18 further comprising a sealed container, wherein said device is substantially contained within said sealed container.

20. An immobilizing device according to Claim 18 further comprising a fastening strap attached to an edge of said first support, said fastening strap having a fastener for securing said first support to the adjacent surface.
